(19) Europäisches Patentamt
European Patent Office
Office européen des brevets

(11) **EP 3 858 407 A1**

(12) **EUROPEAN PATENT APPLICATION**
published in accordance with Art. 153(4) EPC

(43) Date of publication:
04.08.2021 Bulletin 2021/31

(51) Int Cl.:
*A61M 16/00* (2006.01)　　　*A61B 5/087* (2006.01)
*A61M 16/10* (2006.01)

(21) Application number: 19865636.5

(22) Date of filing: 27.09.2019

(86) International application number:
PCT/JP2019/038394

(87) International publication number:
WO 2020/067526 (02.04.2020 Gazette 2020/14)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 28.09.2018 JP 2018185714

(71) Applicant: Teijin Pharma Limited
Tokyo 100-0013 (JP)

(72) Inventors:
• YAMAURA, Yuki
Tokyo 100-0013 (JP)
• MORISHITA, Yuta
Tokyo 100-0013 (JP)
• YOSHIZAWA, Akira
Tokyo 100-0013 (JP)

(74) Representative: Hoffmann Eitle
Patent- und Rechtsanwälte PartmbB
Arabellastraße 30
81925 München (DE)

(54) **RESPIRATION MONITORING DEVICE**

(57) Provided is a respiratory monitoring device that measures and outputs the substantial use time of an oxygen supply device. A respiratory monitoring device (4) used in combination with an oxygen supply device (1) delivering highly concentrated oxygen gas includes a detection unit (6) that detects a change in breathing-related information representing at least one of a pressure, a flow rate, and a gas temperature, based on exhalation and inhalation, a calculation unit (725) that calculates a respiratory rate, based on the change in breathing-related information, a determination unit (726) that determines whether breathing is present, and whether a user of the oxygen supply device is present, based on the respiratory rate, a measurement unit (727) that measures a duration in which a user of an oxygen supply device has been determined to be present, based on a determination result obtained by the determination unit, and an output unit (728) that outputs a cumulative measurement result for the duration.

FIG. 2

**Description**

FIELD

The present disclosure relates to a respiratory monitoring device that is used in combination with an oxygen supply device, measures the substantial use time of the oxygen supply device, and performs output, transmission, display, or recording.

BACKGROUND

Oxygen therapy has been conventionally conducted as one medical treatment for patients suffering from respiratory diseases such as asthma and chronic obstructive pulmonary disease. In this therapy, oxygen having a concentration higher than that of indoor air is administered to the patients. In recent years, HOT (Home Oxygen Therapy) in which oxygen therapy is practiced, e.g., at home or in facilities, aiming to improve the patients' QOL (Quality of Life), is becoming mainstream, and an oxygen concentration device is mainly used as an oxygen source.

Oxygen therapy may be preferably conducted under conditions based on doctor's instructions as an oxygen flow rate and inhalation time are prescribed by doctor's diagnosis. It may suffice to monitor whether oxygen therapy is conducted in accordance with the instructions.

As a method for obtaining the respiratory rate of a patient, which can be implemented in an oxygen supply device, a method is available for mounting a micro-differential pressure sensor for respiratory measurement between an oxygen concentration device and a cannula fitted to the patient to measure a patient respiratory pressure during oxygen inhalation, and recording the patient respiratory pressure on a recording medium or transmitting it as communication data, as disclosed in PTL 1.

PTLs 2 and 4 disclose the fact that the respiratory rate and the like can be calculated from a respiratory pattern, but they describe no specific methods.

PTL 3 discloses a method for storing the timings at which the pressure waveform changes from a fall to a rise and counting the respiratory rate, based on the interval between these timings, and a method for multiplying the amplitude of the pressure by a predetermined detection level ratio and determining that breathing is present only when a threshold corresponding to the calculated product is exceeded.

PTL 5 discloses a method for calculating the respiratory rate by FFT (Fast Fourier Transform) processing or TDS processing.

PTL 6 discloses a method for measuring and storing pressure variations of an oxygen concentration device itself in advance, and subtracting the pressure variations from a detected pressure waveform.

[CITATION LIST]

[PATENT LITERATURE]

[PTL 1] Japanese Unexamined Patent Publication (Kokai) No. H6-190045
[PTL 2] Japanese Unexamined Patent Publication (Kokai) No. 2001-286566
[PTL 3] Japanese Unexamined Patent Publication (Kokai) No. H7-96035
[PTL 4] Japanese Unexamined Patent Publication (Kokai) No. 2015-85191
[PTL 5] Japanese Unexamined Patent Publication (Kohyo) No. 2011-518016
[PTL 6] International Publication No. WO 2018-180392

SUMMARY

For respiratory monitoring of the patient, in any method for directly sending the calculated respiratory data, since waveform data measured every 100 milliseconds (ms), for example, is directly transmitted, the amount of data is enormous, and it may take a long time for analysis.

The oxygen supply device normally includes an extension tube. The extension tube is believed to exert no influence on the flow rate and the oxygen concentration as long as it has a length of about 15 to 20 m or less. During home oxygen therapy, the patient may not always be able to live at rest at all times. When, for example, the tube portion delivering highly concentrated oxygen gas to the patient oscillates upon, e.g., walking of the patient, the method for directly sending the calculated respiratory data may not allow detection of breathing, so a time shorter than an actual inhalation time is counted as the inhalation time in this method. In the method for directly sending the calculated respiratory data, a content that does not satisfy the inhalation time prescribed for the patient may be recorded or the like, and no substantial use time may be counted.

A respiratory monitoring device has been invented to solve the above-described problems, and has as its exemplary object to make it possible to measure and output the substantial use time of an oxygen supply device.

A respiratory monitoring device according to one aspect of an embodiment is provided as a respiratory monitoring device used in combination with an oxygen supply device delivering highly concentrated oxygen gas, the respiratory monitoring device including a detection unit that detects a change in breathing-related information representing at least one of a pressure, a flow rate, and a gas temperature, based on exhalation and inhalation, a calculation unit that calculates a respiratory rate, based on the change in breathing-related information, a determination unit that determines whether breathing is present, and whether a user of the oxygen supply device is present, based on the respiratory rate, a measurement unit that measures a duration in which a user of an oxygen supply device has been determined to be present, based on a determination result obtained by the determination unit, and an output unit that outputs a cumulative measurement result for the duration.

In the respiratory monitoring device according to another aspect of the embodiment, preferably, the determination unit determines that a user of an oxygen supply device is present unless the respiratory rate is zero or is not calculable, and further determines that breathing is present when the respiratory rate is calculated to fall within a predetermined range.

In the monitoring device according to still another aspect of the embodiment, preferably, the predetermined range is set to 8 to 50 bpm.

The respiratory monitoring device according to still another aspect of the embodiment preferably further includes a display unit configured to display, on an identical screen, an operation time of the oxygen supply device and the cumulative measurement result for the duration.

In the respiratory monitoring device according to still another aspect of the embodiment, preferably, the oxygen supply device is implemented as an oxygen concentration device.

In the respiratory monitoring device according to still another aspect of the embodiment, preferably, the detection unit detects a change in pressure based on exhalation and inhalation, and the calculation unit calculates the respiratory rate, based on the change in pressure.

In the respiratory monitoring device according to still another aspect of the embodiment, preferably, the calculation unit calculates the respiratory rate using data having a pressure variation component, independent of exhalation and inhalation, removed from the change in pressure based on the exhalation and inhalation.

In the respiratory monitoring device according to still another aspect of the embodiment, preferably, the calculation unit estimates a pressure variation component based on an operation of the oxygen supply device, on the basis of the change in pressure based on the exhalation and inhalation, and calculates the respiratory rate using the data having the pressure variation component removed from the change in pressure based on the exhalation and inhalation.

According to this embodiment, a respiratory monitoring device that measures and outputs the substantial use time of an oxygen supply device can be provided.

The objects and effects of the present invention will be appreciated and obtained by means of the elements and combinations particularly pointed out in the appended claims. Both the foregoing general description and the following detailed description are exemplary and explanatory, and do not limit the present invention described in the scope of claims.

BRIEF DESCRIPTION OF DRAWINGS

Fig. 1 is a diagram illustrating an exemplary schematic configuration of a PSA oxygen concentration device.

Fig. 2 is a schematic diagram schematically illustrating an exemplary respiratory monitoring device.

Fig. 3 is a block diagram illustrating an exemplary microcomputer unit.

Fig. 4 is a chart representing pressure data including patient respiratory information, under the conditions in which continuous flow is set at 5 LPM, and an extension tube is added by 20 m.

Fig. 5 is a chart representing PSA pressure data extracted by an arithmetic operation unit, under the conditions in which continuous flow is set at 5 LPM, and an extension tube is added by 20m.

Fig. 6 is a chart representing patient respiratory information after subtraction processing, under the conditions in which continuous flow is set at 5 LPM, and an extension tube is added by 20 m.

Fig. 7 is a conceptual diagram depicting the principle of separating patient respiratory information and a PSA pressure from pressure data including the PSA pressure and pressure variations caused by patient breathing.

Fig. 8 is a graph representing the result of calculating an autocorrelation coefficient from the patient respiratory information after the subtraction processing.

Fig. 9 is a flowchart illustrating exemplary processing of extracting patient respiratory information data.

Fig. 10 is a flowchart illustrating exemplary processing of estimating the respiratory rate, based on the patient respiratory information data.

Fig. 11 is a flowchart illustrating exemplary respiratory monitoring processing, based on the respiratory information data.

Fig. 12 is a view illustrating an exemplary respiratory monitoring display screen.

DESCRIPTION OF EMBODIMENTS

A respiratory monitoring device according to one aspect of the present disclosure will be described below with reference to the drawings. However, it should be noted that the technical scope of the present disclosure is not limited to such embodiments and encompasses the invention described in the scope of claims and its equivalents. In the following description and drawings, the same reference numerals denote components having the same functional configurations, and a repetitive description thereof will not be given.

<PSA Oxygen Concentration Device>

A PSA (Pressure Swing Adsorption) oxygen concentration device (to be referred to as a "PSA oxygen concentration device" hereinafter) serving as an exemplary oxygen supply device used in combination with the respiratory monitoring device according to this embodiment will be described below.

The oxygen concentration device means a device that concentrates and delivers oxygen existing at about 21% in the air. Most oxygen concentration devices are generally of the pressure swing adsorption type (to be referred to as the PSA type hereinafter).

As the oxygen concentration devices, the PSA type and the VPSA (Vacuum Pressure Swing Adsorption) type are available. The PSA type refers to a pressure swing adsorption method for depressurization to atmospheric pressure in a desorption process, and the VPSA type refers to a pressure swing adsorption method for depressurizing an adsorption column to vacuum pressure using a compressor to enhance the regeneration efficiency of an adsorbent. In this embodiment, the PSA oxygen concentration device will be taken as an example, but the VPSA oxygen concentration device may be used. Other types of oxygen concentration devices may even be used.

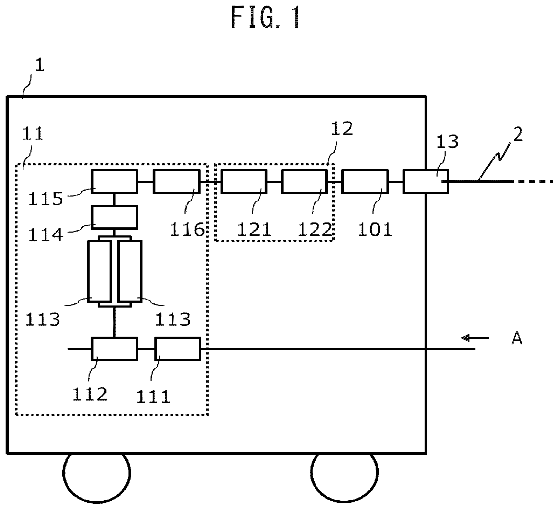

Fig. 1 is a diagram illustrating an exemplary schematic configuration of a PSA oxygen concentration device.

A PSA oxygen concentration device 1 includes an oxygen generation unit 11 that generates concentrated oxygen gas by introducing air A from the exterior of the PSA oxygen concentration device 1.

The air A introduced from the exterior of the PSA oxygen concentration device 1 into the oxygen generation unit 11 is compressed by a compressor 111 and fed into an adsorption column 113 via a first switching valve 112. The first switching valve 112 connects the compressor 111 to one of adsorption columns 113 to feed the compressed air into the connected adsorption column 113, and opens the remaining adsorption columns to the atmosphere.

The adsorption columns 113 are filled with adsorbents for selectively adsorbing nitrogen gas. The compressed air having passed through the adsorption column 113 reduces in nitrogen gas concentration and thus turns into concentrated oxygen gas. The concentrated oxygen gas is stored in a concentrated oxygen buffer tank 115 via a second switching valve 114. The second switching valve 114 connects or disconnects one of the adsorption columns 113 to or from the concentrated oxygen buffer tank 115.

The oxygen generation unit 11 uses the first switching valve 112 to connect the compressor 111 to one of the adsorption columns 113, and uses the second switching valve 114 to connect the adsorption column 113 connected to the compressor 111 to the concentrated oxygen buffer tank 115. Therefore, the compressor 111, one of the adsorption columns 113, and the concentrated oxygen buffer tank 115 are connected to each other, and generated concentrated oxygen gas is supplied to the concentrated oxygen buffer tank 115. The adsorption columns 113 that are not connected to the compressor 111, however, are opened to the atmosphere via the first switching valve 112, as they are disconnected from the concentrated oxygen buffer tank 115 by the second switching valve 114. With this operation, the adsorption columns 113 are depressurized to exhaust the nitrogen gas adsorbed to the adsorbents out of the PSA oxygen concentration device 1.

Opening and closing of the first switching valve 112 and the second switching valve 114 are controlled by, e.g., a microcomputer unit in a respiratory monitoring device (not illustrated). The microcomputer unit can acquire a timing to switch between pressurization and depressurization in the adsorption columns 113. The respiratory monitoring device may be placed in the oxygen concentration device 1, or may be placed outside the oxygen concentration device 1 separately from the oxygen concentration device 1.

As another example, the oxygen concentration device 1 may include an oxygen concentration control unit that controls oxygen concentration processing including processing of opening and closing the first and second switching valves. The microcomputer unit can acquire a timing to switch between pressurization and depressurization in the adsorption columns 113 from the oxygen concentration control unit.

The PSA oxygen concentration device 1 may connect two or more adsorption columns of the adsorption columns 113, in addition to the above-mentioned basic configuration. The PSA oxygen concentration device 1 may include an additional process such as a pressure equalization process of equalizing the pressures in the respective adsorption columns, or a purge process of refluxing part of the generated concentrated oxygen gas to one of the adsorption columns 113.

Normally, after the compressed air is fed into the adsorption column 113, the adsorption column 113 is opened to the atmosphere as it is disconnected from the compressor 111 by the first switching valve 112. In contrast to this, any adsorption column 113 opened to the atmosphere is connected to the compressor 111 by the first switching valve 112, and shifts to the process of oxygen compression. In this manner, the use of the first switching valve 112 to allow the adsorption columns 113 to alternately repeat compression and opening to the atmosphere makes it possible to continuously supply concentrated oxygen gas.

Since the pressure change in the adsorption column 113 upon concentrated oxygen generation is very large, periodical pressure variations occur in the pressure within an oxygen gas flow path, formed downstream of the adsorption column 113, upon switching of the adsorption column 113. The concentrated oxygen gas stored in the concentrated oxygen buffer tank 115 is regulated to attenuate the pressure variations by a pressure regulating valve 116.

The concentrated oxygen gas having its pressure regulated by the oxygen generation unit 11 has its oxygen flow rate controlled by an oxygen flow control unit 12 formed by a control valve 121 and a flowmeter 122, and is supplied to the exterior of the oxygen concentration device by an oxygen supply port 13 via a humidifier 101. In the oxygen flow control unit 12, either the control valve 121 or the flowmeter 122 may be provided upstream in the flow path, and the oxygen flow control unit 12 may include other configurations or arrangements.

The PSA oxygen concentration device may include a switching fixed orifice for switching the flow rate, in place of the flowmeter 122 and the control valve 121. The PSA oxygen concentration device 1 may also use a scheme of manually controlling the flow rate using a visually-observable flowmeter such as a rotor meter as the flowmeter 122, and a manual flow control valve in place of the control valve 121, or may use other flow control methods. The PSA oxygen concentration device can even have a configuration equipped with no humidifier 101.

<Respiratory Monitoring Device>

An exemplary schematic configuration of a respiratory monitoring device according to this embodiment will be described below.

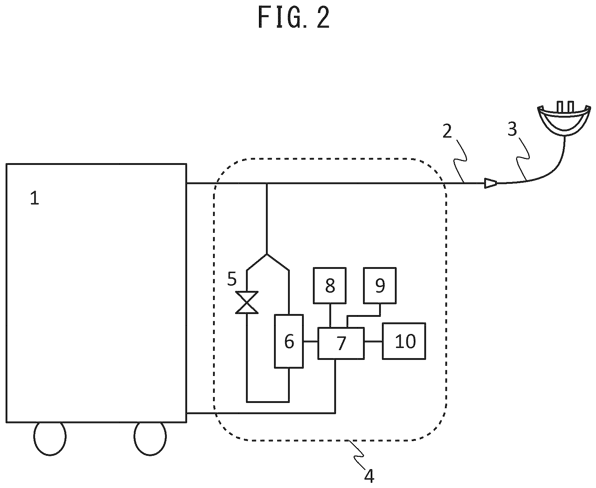

Fig. 2 is a diagram illustrating an exemplary schematic configuration of a respiratory monitoring device according to the present invention.

Oxygen generated by the PSA oxygen concentration device 1 is supplied to the nasal cavity of a patient, acting as a user, via a tube 2 connected to the PSA oxygen concentration device 1, and a nasal cannula 3 connected to the tube 2. The patient constantly breathes even during oxygen inhalation, and a pressure change occurring upon the breathing of the patient propagates toward the nasal cannula 3, the tube 2, and the PSA oxygen concentration device 1.

In this embodiment, to monitor the state in which the patient acting as the user uses the PSA oxygen concentration device 1, a respiratory monitoring device 4 is connected to the tube 2 serving as an oxygen supply path. The tube refers to one including the entire tube provided between the humidifier 101 (the oxygen flow control unit 12 when the PSA oxygen concentration device 1 is equipped with no humidifier 101) and the nasal cannula 3, and the respiratory monitoring device 4 may be connected anywhere in the tube.

Hence, a part or the whole of the respiratory monitoring device 4 may be placed either inside or outside the PSA oxygen concentration device 1.

The inventors of the present invention made a close study, and discovered that even after the pressure variations are attenuated by the pressure regulating valve 116, pressure variations applied to the respiratory monitoring device 4 include those occurring upon pressurization and depressurization during concentrated oxygen gas generation. Since the amplitude of the pressure variations occurring upon this concentrated oxygen gas generation is larger than that of the respiratory pressure, and the amplitude of the respiratory pressure also reduces due to a pressure loss produced upon passage through the oxygen flow path, it is difficult to directly measure the respiratory pressure waveform of the patient from the pressure measured by the respiratory monitoring device 4.

In this embodiment, the respiratory monitoring device 4 includes a microcomputer unit 7 that performs processing of calculating the respiratory rate of the patient and is connected to a pressure sensor 6. The pressure sensor 6 is preferably implemented as a micro-differential pressure sensor. The microcomputer unit 7 further performs processing of measuring and outputting, based on the calculated respiratory rate, the presence or absence of breathing, and the duration in which the user of the PSA oxygen concentration device 1 has been determined to be present. The microcomputer unit 7 is connected to the PSA oxygen concentration device 1.

The respiratory monitoring device 4 further includes a display unit 8, such as a liquid crystal display, that is connected to the microcomputer unit 7, and displays the respiratory rate, the presence or absence of breathing, and the duration in which the user of the PSA oxygen concentration device 1 has been determined to be present.

The respiratory monitoring device 4 preferably further includes a transmission unit 9 that is connected to the microcomputer unit 7, and transmits to the exterior information concerning the respiratory rate, the presence or absence of breathing, and the duration in which the user of the PSA oxygen concentration device 1 has been determined to be present. The transmission unit 9 is implemented as, e.g., Wi-Fi, Bluetooth (registered trademark), or other wireless communication modules.

The respiratory monitoring device 4 preferably further includes an external storage unit 10 that is connected to the microcomputer unit 7, and stores, on an external storage medium, the respiratory rate, the presence or absence of breathing, and the duration in which the user of the PSA oxygen concentration device 1 has been determined to be present. The external storage unit 10 is implemented as, e.g., an external hard disk, a flash memory R/W unit, or a DVD drive.

Each of the display unit 8, the transmission unit 9, and the external storage unit 10 is connected to the microcomputer unit 7 and controlled by the microcomputer unit 7.

In this embodiment, the respiratory monitoring device 4 includes, e.g., a pressure sensor 6, and preferably a micro-differential pressure sensor 6, as a detection unit that detects and outputs the pressure in the tube, and a microcomputer unit 7 electrically connected to the detection unit. The respiratory monitoring device 4 may further include a positive-displacement unit connected to the micro-differential pressure sensor 6, and a pressure smoothing unit formed by an orifice 5 connecting the tube and the positive-displacement unit to each other. The reason why the pressure smoothing unit is provided is as follows.

Since the respiratory pressure of the patient is normally about $\pm 10$ to 100 Pa, a sensor having the range of about $\pm 100$ Pa is preferably used as the micro-differential pressure sensor 6 to obtain the respiratory pressure by the respiratory monitoring device 4. In the state in which oxygen is supplied from the oxygen concentration device, a supply pressure is constantly generated upon oxygen supply, and the supply pressure generated upon oxygen supply exists at, e.g., about 300 Pa even for 1 LPM (litter per minute: l/min). This means that when one end of the micro-differential pressure sensor 6 is connected to the oxygen supply path, as described above, with the other end of the micro-differential pressure sensor 6 being opened to the atmosphere, the pressure to be measured falls outside the measurement range of the micro-differential pressure sensor 6. Therefore, a pressure including respiratory information of the patient is preferably obtained in the measurement range of the micro-differential pressure sensor 6 by applying the pressure after passage through the orifice 5 to the other end of the micro-differential pressure sensor 6.

A pressure that falls within the measurement range of the micro-differential pressure sensor 6 may be preferably applied to the other end of the micro-differential pressure sensor 6, and a method for this operation is not limited to the example in this embodiment. As long as the measurement range of the micro-differential pressure sensor 6 is higher than the supply pressure generated upon oxygen supply, and a resolution high enough to allow detection of pressure variations occurring upon breathing of the patient can be ensured, the other end of the micro-differential pressure sensor 6 may even be opened to the atmosphere, and a pressure sensor that measures a gauge pressure or an absolute pressure may be substituted for the micro-differential pressure sensor.

Since a pressure change upon breathing also appears as minute flow rate variations of the concentrated oxygen gas flowing through the tube, a flow sensor may be substituted for the pressure sensor 6. The pressure sensor 6 and/or the flow sensor serves as a detection unit that detects an in-tube pressure and/or an in-tube gas flow rate in the tube 2 including respiratory information of the patient, and outputs pressure data and/or in-tube gas flow rate data.

<Microcomputer Unit>

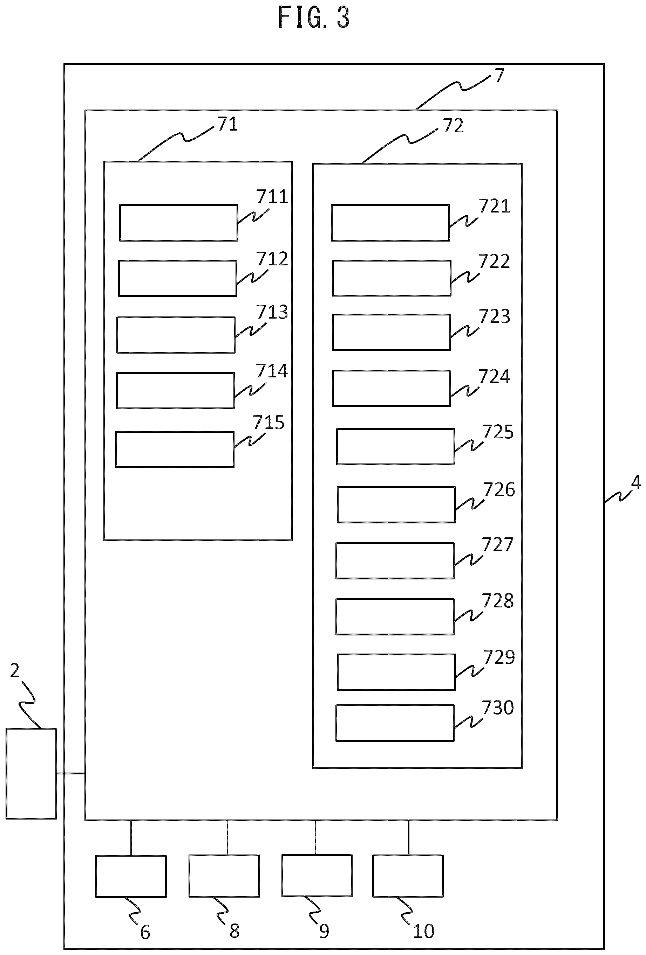

Fig. 3 is a diagram illustrating an exemplary configuration block of the microcomputer unit 7.

The microcomputer unit 7 including an arithmetic operation unit 722 and an estimation unit 723 is connected to the pressure sensor, and preferably the micro-differential pressure sensor 6. The arithmetic operation unit 722 and the estimation unit 723 obtain respiratory information of the patient by receiving the in-tube pressure data and or the in-tube gas flow rate data in the tube including the respiratory information of the patient, detected by the micro-differential pressure sensor 6 serving as the detection unit, and performing processing involved (to be described later).

The microcomputer unit 7 may be implemented as the same microcomputer as in a processing unit that processes, e.g., an oxygen generation function and a display and user interface function for the oxygen concentration device, or may be separate from the processing unit. When the microcomputer unit 7 is separate from the processing unit, it acquires a timing to switch a PSA period T from a microcomputer that processes the oxygen generation function, and uses the acquired timing for arithmetic operation.

The microcomputer unit 7 includes a storage unit 71 and a processing unit 72. The storage unit 71 is implemented as one or more semiconductor memories. The storage unit 71 includes at least one of nonvolatile memories such as a RAM, a flash memory, an EPROM, and an EEPROM. The storage unit 71 stores, e.g., a driver program, an operating system program, an application program, and data used for processing by the processing unit 72.

The storage unit 71 stores, as the driver program, e.g., a device driver program for controlling, e.g., the micro-differential pressure sensor 6 serving as the detection unit. A computer program may be installed in the storage unit 71 using, e.g., a known setup program from a computer-readable portable recording medium such as a CD-ROM or a DVD-ROM. The computer program may even be downloaded from, e.g., a program server and installed.

The storage unit 71 may further temporarily store temporary data associated with predetermined processing. The storage unit 71 stores, e.g., a threshold 711, a variation value data file 712, a cumulative measurement result data file 713, an operation data file 714, a monitoring display image 715, and various other thresholds for use in estimation of the respiratory rate.

The processing unit 72 includes one or more processors and their peripheral circuits. The processing unit 72 systematically controls the overall operation of the respiratory monitoring device 4, and is implemented as, e.g., an MCU (Micro Control Unit).

The processing unit 72 performs processing based on the programs (e.g., the operating system program, the driver program, and the application program) stored in the storage unit 71. The processing unit 72 may execute several programs (e.g., the application program) in parallel. The processing unit 72 includes a detected data acquisition unit 721, the arithmetic operation unit 722, the estimation unit 723, a respiratory rate output unit 724, a calculation unit 725, a determination unit 726, a measurement unit 727, an output unit 728, a display control unit 729, and an operation data acquisition unit 730.

Each of these units constituting the processing unit 72 may be implemented in the microcomputer unit 7 as an independent integrated circuit, circuit module, microprocessor, or firmware.

<Principle of Arithmetic Processing Performed in Embodiment>

The principle of processing performed in this embodiment, using data of respiratory information obtained by applying a respiratory pressure in a patient respiratory model from the nasal cannula 3 using the configuration illustrated in Fig. 2, will be described below.

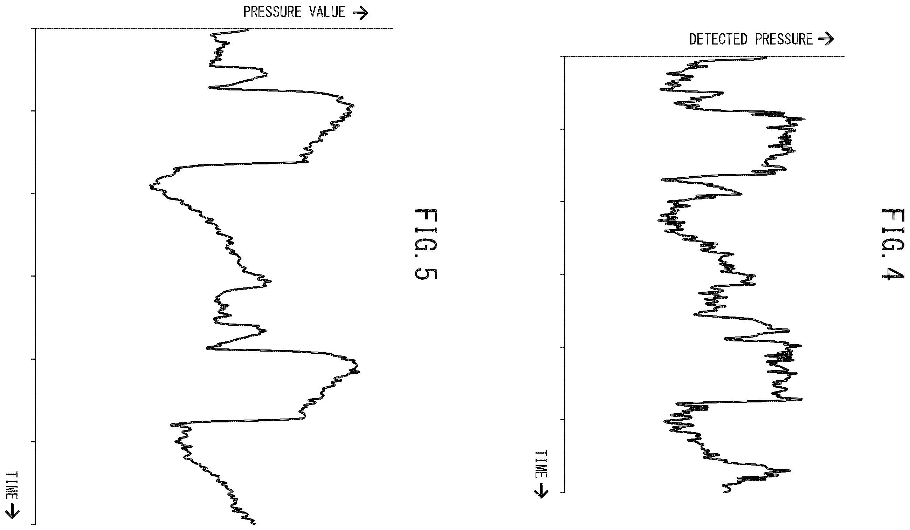

Figs. 4, 5, and 6 illustrate a group of data obtained when continuous flow is set at 5 LPM, and a 20-m extension tube is connected on the downstream side of the respiratory monitoring device 4. The continuous flow means one concentrated oxygen gas supply scheme, in which concentrated oxygen gas is continuously supplied at a constant flow rate.

Fig. 4 is a chart representing pressure data including respiratory information of the patient and PSA pressure data of the PSA oxygen concentration device 1, when continuous flow is set at 5 LPM, and a 20-m extension tube is connected.

Fig. 5 is a chart representing PSA pressure data of the PSA oxygen concentration device 1 if the patient is not breathing or after respiratory components are removed, when continuous flow is set at 5 LPM, and a 20-m extension tube is connected.

The PSA pressure obtained by the PSA oxygen concentration device means a periodical pressure change related to the adsorption column period of the PSA oxygen concentration device 1 and occurring when oxygen is generated by the PSA oxygen concentration device 1, as described above. Therefore, the period of the waveform of the PSA pressure coincides with the adsorption column switching period of the PSA oxygen concentration device 1.

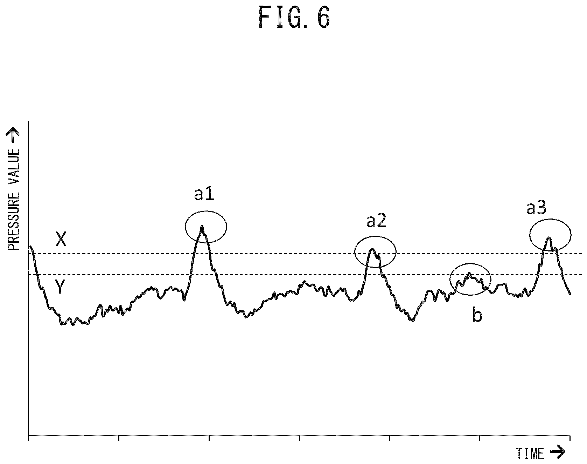

Fig. 6 is a chart representing the result of subtraction processing of components illustrated in Fig. 5 from components illustrated in Fig. 4 by software. The subtraction processing means processing of computing the difference between two pieces of data at an arbitrary time instant. The subtraction processing can remove PSA pressure components and detect a respiratory pressure in a patient respiratory model. In this manner, by performing the subtraction processing by software, the respiratory monitoring device 4 can obtain respiratory information of the patient even in the state in which oxygen is continuously inhaled at 5 LPM from the PSA oxygen concentration device 1 via the nasal cannula and the 20-m extension tube.

In extracting patient respiratory information data by removing variation value data such as the PSA pressure components, the method for measuring and storing pressure variations of an oxygen concentration device itself in advance, and subtracting the pressure variations from a detected pressure waveform, as disclosed in PTL 6, may be used, or a method for subtracting pressure variations of an oxygen concentration device itself measured in real time from a detected pressure waveform may be used, as will be described in detail below.

When the PSA oxygen concentration device switches between pressurization and depressurization with a single period T during at least a part of the total operation time, the arithmetic operation unit 722 extracts respiratory information by calculating, as the pressure at a certain time t, the difference between a flow rate value $Y(t)$ and a value $X(t)$ obtained by averaging $Y(t)$ and $Y(t - T)$, $Y(t - 2T)$,..., $Y(t - nT)$ (n is a preset arbitrary integer). An original respiratory waveform having the PSA pressure components removed can thus be accurately reproduced.

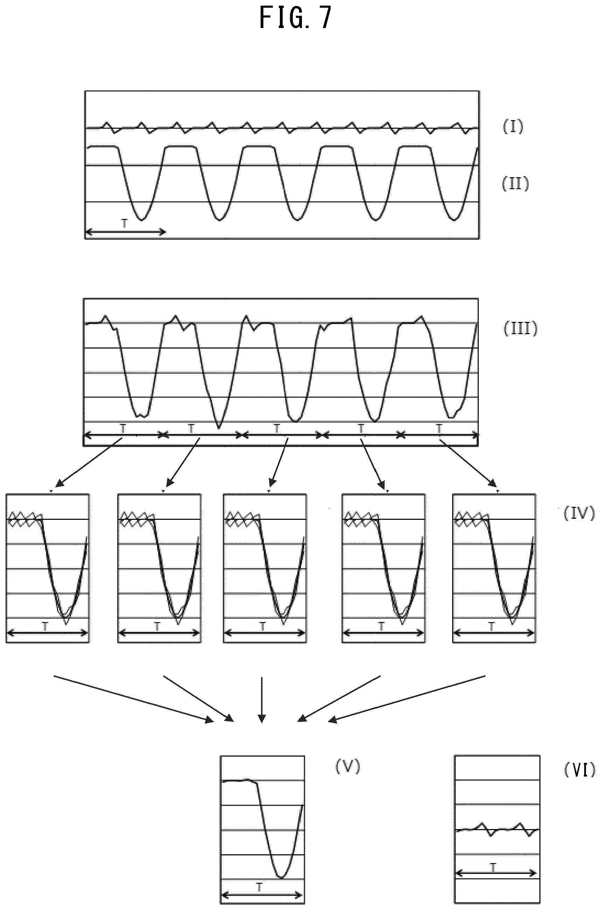

Fig. 7 is a conceptual diagram depicting the above-mentioned arithmetic processing. (I) illustrates a respiratory waveform, and (II) illustrates a model waveform for the PSA waveform. T denotes the period of the PSA waveform, which coincides with the switching period of the adsorption columns. (III) illustrates the sum of the waveforms illustrated in (I) and (II), and corresponds to the pressure measured in the tube portion. (IV) illustrates the result of dividing the waveform of (III) for each period T and superimposing the divided waveforms on each other. Unless the respiratory period and the PSA period completely coincide with each other, respiratory waveforms randomly appear in the waveforms divided by the periods T. (V) illustrates the result of averaging the waveforms superimposed on each other in (IV). In other words, based on the measured pressure data and/or gas flow rate data (III), variation value data X(t) representing a periodical pressure change and/or flow rate change of concentrated oxygen gas generated by the operation of the PSA oxygen concentration device is estimated by superimposition and averaging (V).

In one example, assuming T as one period, a simple moving average of five periods is calculated. (VI) illustrates a waveform obtained by subtracting the waveform of (V) from the portion corresponding to the last period T of the waveform of (III). This reveals that the original respiratory waveform (I) can be accurately reproduced. As an exemplary period T, the time from the start of the state in which one of the adsorption columns is connected to the compressor until a shift is made to the state in which another adsorption column is connected to the compressor may be selected. As another exemplary period T, the time from the start of the state in which one adsorption column is connected to the compressor, and then through the state in which the remaining adsorption columns are connected to the compressor, until a shift is made to the state in which the first adsorption column is connected to the compressor again may be selected. As still another exemplary period T, a multiple of each switching time may be selected.

In computing the waveform of (V) representing variation value data X(t) obtained by superimposition and averaging and estimated to bear the information of a periodical pressure change and/or flow rate change of the concentrated oxygen gas, a moving average of five periods is calculated in the above description, but another method may be used for averaging. The average value X(t) computed in the step of (V) is generally given by the following equation:
[Math. 1]

$$X(t) = \frac{\sum_{i=0}^{n} a_i Y(t - iT)}{\sum_{i=0}^{n} a_i} \quad \cdots \quad (1)$$

Throughout the entire description of the present disclosure, i does not represent an imaginary number, but it simply represents an integer variable i.

Note that X(t) is the value after averaging processing at time t, and Y(t) is the actual measured value of the pressure at time t. $a_i$ is a weighting coefficient in weighted averaging, which can be obtained by selecting an arbitrary real number, n is the number of values of Y to be averaged. For, e.g., n = 4, and $a_0$ to $a_4$ = 1, the same computation as in Fig. 6 is performed. For $a_i = e^{-bi}$ (b is an arbitrary positive real number), and n = ∞, exponential smoothing is applicable.

Since it is difficult to handle infinity in actual computation, an asymptotic value is obtained for X(t) by sequential computation as, e.g., X(t) = (1 - $e^{-b}$)Y(t) + $e^{-b}$X(t - T). Appropriately selecting n and $a_i$ also allows averaging processing that achieves more rapid convergence, such as an FIR (Finite Impulse Response) filter.

Equation (1) is apparently seen as an equation for simply obtaining the time average of Y(t) by numerical computation. In normal time averaging, a value sufficiently smaller than the variation period of Y(t) is selected as T, while in this embodiment, it is important to select, as T, a value based on the adsorption and desorption period of a PSA process. This means, instead of simply temporally averaging the measured values Y(t), using the waveform of Y(t) over the period T as one unit to calculate the average of waveforms obtained for a duration of an integer multiple of T preceding a certain time of reference. Calculating the average of the waveforms makes it possible to accurately estimate a PSA pressure having characteristics periodically appearing at an interval equal to T.

In this example, a method for measuring the pressure has been exemplified as the respiratory rate measurement method, but since the rate of flow through the tube also changes in response to the pressure variations in practice, a method for measuring the flow rate in place of the pressure can also be used. A method for measuring a pressure value and a flow rate value in combination or selectively, in accordance with the device operation conditions or environmental conditions, can even be used.

The respiratory information obtained by arithmetic processing is so-called raw data, which represents real-time information of breathing such as the pressure or the flow rate in the form of a waveform. This data may be directly recorded or transmitted, but in this case, the amount of data is enormous, and it takes a long time for analysis. It is, therefore, desired to automatically calculate respiratory rate data in the respiratory monitoring device 4, and then record and/or transmit it.

As exemplified above, a detectable respiratory waveform contains a large number of noise components generated upon, e.g., airflow-related pressure variations or cannula oscillation. The respiratory period and the respiratory rate may not be appropriately calculated either by peak detection for calculating the timings at which the pressure changes from a decrease to an increase, or by a method for detecting the timings at which a value equal to or larger than a threshold has been reached.

Referring to, e.g., Fig. 6, respiratory waveforms to be counted appear as peaks indicated by a1, a2, and a3 in Fig. 6 and valleys appearing immediately after these peaks, and the remaining portions correspond to the noise components. Even when one attempts to detect these peaks as the timings at which the pressure changes from an increase to a decrease, it is highly probable that a portion indicated by b in Fig. 6 will be detected.

<Respiratory Rate Estimation Processing>

Even with a method for determining a portion having a value equal to or larger than a certain threshold as a peak, when the threshold is set to X, a peak as low as that indicated by a2 may fail to be detected, and when the threshold is set to Y, the noise component indicated by b may be detected as a peak. In this case, a waveform corresponding to only about three breaths is illustrated, but in an actual waveform, the levels of peaks or noise components may vary more seriously than those illustrated herein, and it is, therefore, very difficult to reliably identify peaks and set a threshold that allows reliable noise removal.

The inventors of the present invention conducted a close examination, and found that the respiratory rate can be detected with high detection performance not by the above-mentioned method, but by calculating an autocorrelation coefficient between an original waveform and a waveform shifted from the original waveform by a time $\Delta t$, and calculating $\Delta t$, in which the autocorrelation coefficient takes a peak, while changing $\Delta t$. These inventors also found a respiratory rate estimation method for estimating the respiratory rate per predetermined time from patient respiratory information.

An autocorrelation coefficient R can be calculated as the following equation:

[Math. 2]

$$R(\Delta t) = \frac{1}{(n - \Delta t)\sigma^2} \sum_{j=1}^{n - \Delta t/t_0} \{f(t - jt_0) - \mu\}[f\{t - (jt_0 + \Delta t)\} - \mu]$$

where $\Delta t$ is the amount of shift in time, $t_0$ is the data acquisition interval, n is the number of data used for one computation operation of the autocorrelation coefficient, and f(t) is the value of respiratory information data at time t, which is acquired n times at the interval $t_0$ in this computation. $\mu$ and $\sigma$ are the average and the standard deviation of f(t), but in actual computation, the average and the standard deviation of the n obtained values of f(t) may be used. The method for calculating the autocorrelation coefficient in this embodiment is merely an example, and other methods for calculating the autocorrelation coefficient may be used.

The autocorrelation coefficient R calculated in this embodiment is multiplied by a normalization coefficient expressed as:

[Math. 3]

$$\frac{1}{(n - \Delta t)\sigma^2}$$

to define its value in the range of -1.0 to +1.0.

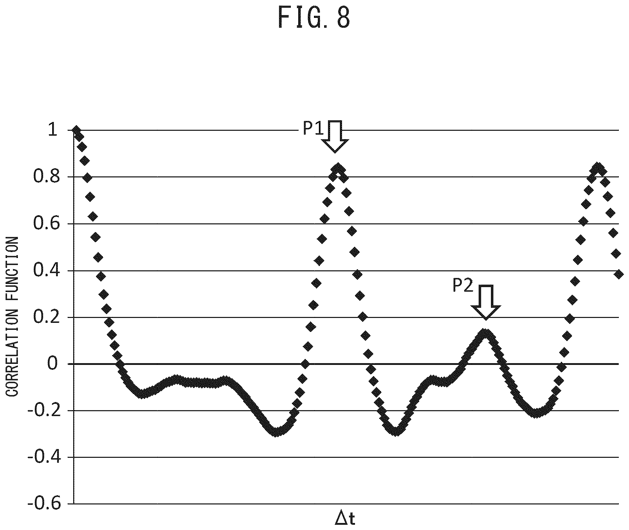

A graph generated by plotting, with respect to $\Delta t$, the computation result of the autocorrelation coefficient between the calculated waveform illustrated in Fig. 6 and the waveform obtained by shifting the original waveform along the time axis by the time $\Delta t$, using the above-mentioned equation multiplied by the normalization coefficient, is obtained as illustrated in Fig. 8. The autocorrelation coefficient is computed every time $\Delta t$ is incremented by $t_0$ from $\Delta t = 0$. When $\Delta t = 0$, the autocorrelation coefficient is 1 because it represents the correlation between the original waveforms. After that, points P1 and P2 are obtained as points having a high correlation.

Fig. 8 reveals that even for a waveform that can hardly be used to determine the respiratory period either based on a threshold or by peak detection in raw respiratory information data, the respiratory period can easily be calculated by calculating the autocorrelation coefficient.

The data interval of f(t) used to compute the autocorrelation coefficient is determined from the range of the respiratory period to be calculated. The inventors of the present invention conducted a close examination, and discovered that the data interval of f(t) may be preferably set twice or more a minimum respiratory period to be calculated. The respiratory rate of an average adult is about 12 to 20 breaths per minute, which corresponds to a respiratory period of about 3 to 5 seconds. These inventors thus concluded that the data interval of f(t) may be preferably set to 5 seconds × 2 = 10 seconds.

It was found that the threshold of the autocorrelation coefficient for determining a peak of the autocorrelation coefficient may be preferably set to at least 0.3, and more preferably set in the range of 0.3 to 0.7. It was also found that setting the threshold below 0.3 increases the probability that an accidental rise in autocorrelation value due to, e.g., noise or baseline instability will be erroneously determined as a peak, and setting the threshold above 0.7 raises the probability that a rise in autocorrelation value corresponding to the respiratory period will be missed.

The respiratory rate is calculated as the reciprocal of the respiratory interval that is the value of $\Delta t$ when the autocorrelation coefficient takes a certain value or more and a peak value. $\Delta t$ at the point P1 that is the first peak in Fig. 8 is determined as the respiratory interval, and dividing one minute by the respiratory interval yields a respiratory rate per minute. The respiratory rate or the number of breaths per minute (bpm) is given by the following equation:

$$\text{Respiratory Rate per Minute (bpm)} = 60 \text{ Seconds}/\Delta t$$

Depending on the respiratory waveform, a plurality of points may be present as peaks having values equal to or larger than a certain value, as illustrated in Fig. 8, but the right peak P2 is a peak corresponding to a multiple period appearing when $\Delta t$ is shifted by several breaths. In estimating the respiratory rate, the peak P1 located leftmost, i.e., having the smallest value of $\Delta t$, which is estimated to correspond to the basic period of breathing, may be preferably used.

By the computation described in this embodiment, respiratory rate information is automatically accurately calculated from the respiratory waveform obtained by the respiratory monitoring device 4 connected to the oxygen concentration device 1.

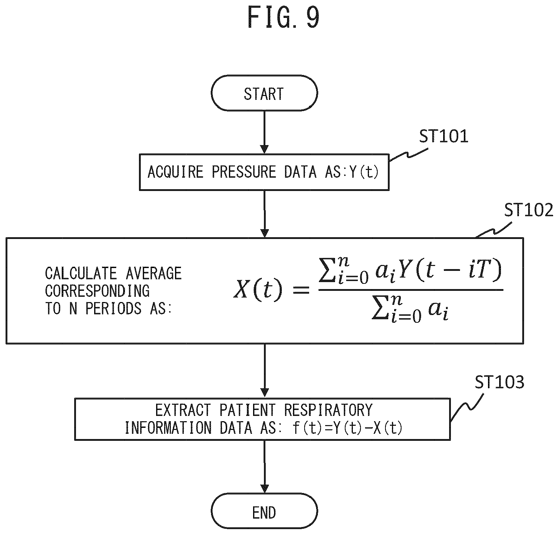

Fig. 9 is a flowchart illustrating exemplary processing of extracting patient respiratory information data.

The processing of extracting patient respiratory information data illustrated in Fig. 9 is performed by the microcomputer unit 7 in accordance with the computer program stored in the storage unit 71 in advance. The detected data acquisition unit 721 acquires pressure data Y(t) from the pressure sensor 6 serving as the detection unit (ST101). The arithmetic operation unit 722 calculates an average X(t) of Y(t) corresponding to n periods (ST102). The arithmetic operation unit 722 extracts patient respiratory information data f(t) by calculating the difference between Y(t) and X(t) (ST103). When PSA pressure variations measured and stored in advance are used, the arithmetic operation unit 722 does not perform the process of ST102, and extracts patient respiratory information data f(t) by calculating the difference between Y(t) and the PSA pressure variations measured and stored in advance, as ST103, after the process of ST101.

During the operation of the respiratory monitoring device 4, processing of estimating the respiratory rate is repeatedly performed, so that patient respiratory information data f(t) is extracted and updated every time, for example, n pieces of measurement data of the pressures Y(t) are acquired at the interval t0. The patient respiratory information data f(t) may even be extracted at a predetermined interval and be updated.

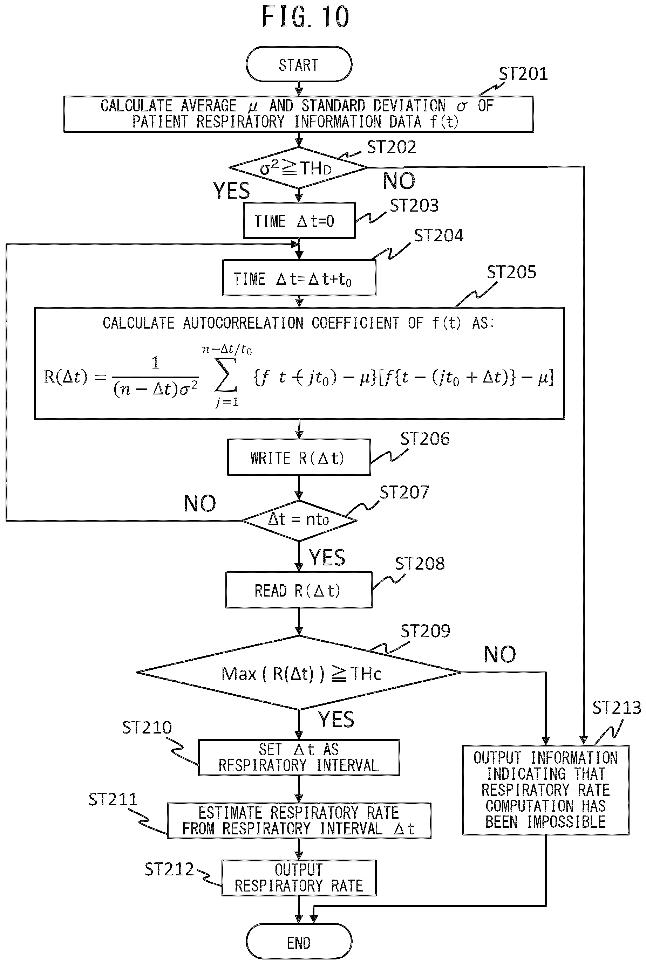

Fig. 10 is a flowchart illustrating exemplary processing of estimating the respiratory rate, based on the patient respiratory information data.

The processing of estimating the respiratory rate illustrated in Fig. 10 is performed by the microcomputer unit 7 in accordance with the computer program stored in the storage unit 71 in advance. The estimation unit 723 calculates an average $\mu$ and a standard deviation $\sigma$ of the patient respiratory information data f(t) (ST201). First, to determine the presence or absence of a respiratory rate, the estimation unit 723 determines whether the variance $\sigma^2$ that is the square of the standard deviation $\sigma$ is equal to or higher than a predetermined threshold $TH_D$ (ST202), as will be described later. When the variance $\sigma^2$ is lower than the threshold $TH_D$ (NO in ST202), the respiratory rate output unit 724 outputs information indicating that computation has been impossible (ST213), and ends the process. When the variance $\sigma^2$ is equal to or higher than the threshold $TH_D$ (YES in ST202), the estimation unit 723 sets the time $\Delta t$ to zero (0) as the amount of shift in time (ST203).

The estimation unit 723 increments the time $\Delta t$ by the data acquisition interval $t_0$ (ST204). The estimation unit 723 calculates an autocorrelation coefficient R($\Delta t$) of the patient respiratory information data f(t) for the time $\Delta t$ (ST205). The estimation unit 723 writes the calculated autocorrelation coefficient R($\Delta t$) into the storage unit 71 (ST206). The estimation unit 723 determines whether the time $\Delta t$ has reached nto (ST207). When the time $\Delta t$ has not reached nto, the process returns to ST203, in which estimation unit 723 repeats a series of processes (NO in ST207).

When the time $\Delta t$ has reached nto (YES in ST207), the estimation unit 723 reads the autocorrelation coefficients R($\Delta t$) stored in the storage unit 71 (ST208). The estimation unit 723 compares the read autocorrelation coefficients R($\Delta t$) with each other to determine whether a maximum autocorrelation coefficient R($\Delta t$), i.e., a peak autocorrelation coefficient R($\Delta t$) is present (ST209). More specifically, the estimation unit 723 determines whether Max(R($\Delta t$)) is equal to or higher than a predetermined threshold $TH_C$.

When the maximum autocorrelation coefficient Max(R(Δt)) is equal to or higher than the predetermined threshold TH$_C$ (YES in ST209), the estimation unit 723 sets the time Δt for the maximum autocorrelation coefficient Max(R(Δt)) as a respiratory interval (ST210). The estimation unit 723 estimates the respiratory rate from the respiratory interval Δt (ST211). The respiratory rate output unit 724 outputs a respiratory rate signal (ST212), and ends the process. For example, the display unit 8, upon receiving the respiratory rate signal, displays the respiratory rate.

When the maximum autocorrelation coefficient Max(R(Δt)) is lower than the predetermined threshold TH$_C$ (NO in ST209), the respiratory rate output unit 724 outputs information indicating that computation has been impossible (ST213), and ends the process. During the operation of the respiratory monitoring device 4, processing of estimating the respiratory rate is repeatedly performed, so that the respiratory monitoring device 4 can update the respiratory rate and display it on the display unit 8. The display on the display unit 8 may be performed at a predetermined interval.

When patient respiratory information data f(t) representing a change in information pertaining to, e.g., the pressure, based on exhalation and inhalation, contains only small noise other than breathing, the autocorrelation coefficient may accidentally become high with a certain period. In view of this, by additionally performing determination based on the variance of the patient respiratory information data f(t), the calculation unit 725 can achieve a higher accuracy by calculating no respiratory rate when no wave having a magnitude high enough to determine that breathing is expected to be included is available. As the determination of the presence or absence of breathing, determination based on the variance of the patient respiratory information data f(t) has been exemplified, but other determination methods may be used.

A variance $\sigma^2$ of the respiratory information data f(t) is calculated as the following equation:

[Math. 4]

$$\sigma^2 = \frac{1}{n} \sum_{t=1}^{n} (f(t) - \mu)^2$$

where n is the number of data used for one computation operation of the autocorrelation coefficient, f(t) is the value of respiratory information data at time t, and $\mu$ and $\sigma^2$ are the average and the variance of f(t).

As one example, the threshold for variance evaluation in the use of the PSA oxygen concentration device is set as follows:

When Respiratory Rate Is Less Than Eight Breaths: $\sigma^2 \geq 18$ (Pa$^2$)
When Respiratory Rate Is Eight to 10 Breaths: $\sigma^2 \geq 18$ (Pa$^2$)
When Respiratory Rate Is 11 Breaths or More: $\sigma^2 \geq 7$ (Pa$^2$)

The value of this threshold, however, may vary depending on the oxygen supply device used. Because of its variations that depend on parameters such as the respiratory rate, the threshold for variance evaluation may be preferably appropriately set in accordance with the oxygen supply device used.

As one example, a first threshold TH$_{D1}$ for the variance $\sigma^2$ of the respiratory information data f(t) when the respiratory rate is less than eight breaths is set to TH$_{D1}$ = 18, a second threshold TH$_{D2}$ for the variance $\sigma^2$ of the respiratory information data f(t) when the respiratory rate is eight to 10 breaths is set to TH$_{D2}$ = 18, and a third threshold TH$_{D3}$ for the variance $\sigma^2$ of the respiratory information data f(t) when the respiratory rate is 11 breaths or more is set to TH$_{D3}$ = 7. When the respiratory information data f(t) is equal to or higher than the threshold of the variance $\sigma^2$, the presence or absence of breathing is determined.

When, in addition to the above-mentioned estimation of the respiratory rate, the substantial use time of the oxygen supply device 1 can be measured, and the measured time can be compared with a prescribed inhalation time, this is beneficial for therapy. In view of this, the respiratory monitoring device 4 performs the following respiratory monitoring processing.

<Respiratory Monitoring Processing>

When the respiratory rate estimated from the respiratory information data f(t) falls within an appropriate range of breathing (e.g., 8 to 50 bpm), the respiratory monitoring device 4 determines that breathing is present, and the oxygen supply device 1 is in use. When the respiratory rate is zero or has not been calculable, the respiratory monitoring device 4 determines that breathing is absent, and the oxygen supply device 1 is not in use. When the respiratory rate is other than zero and falls outside the appropriate range of breathing (e.g., less than 8 bpm, or more than 50 bpm), the respiratory monitoring device 4 determines that breathing is absent, and the oxygen supply device 1 is in use.

The time measured when the oxygen supply device 1 has been determined to be in use is defined as an estimated inhalation time. By consequentially taking into account not only the time measured when breathing has been determined to be present, but also the time measured when the oxygen supply device 1 has been determined to be in use, the accumulated estimated inhalation time is defined as the substantial use time of the oxygen supply device 1.

Table 1 represents the above-mentioned respiratory rate determination processing.

[Table 1]

| Autocorrelation Coefficient of Respiratory Information | Less than Threshold | Threshold or More | | | |
|---|---|---|---|---|---|
| Variance of Respiratory Information | - | Less than Threshold | Threshold or More | | |
| Respiratory Rate Provisionally Calculated from Respiratory Information | Not Calculable | Not Calculable | Less than 8 | 8 to 50 | More than 50 |
| Accumulation on Estimated Inhalation Time | Not Done | Not Done | Done | Done | Done |
| Respiratory Rate | Invalid | Invalid | Invalid | Valid | Invalid |

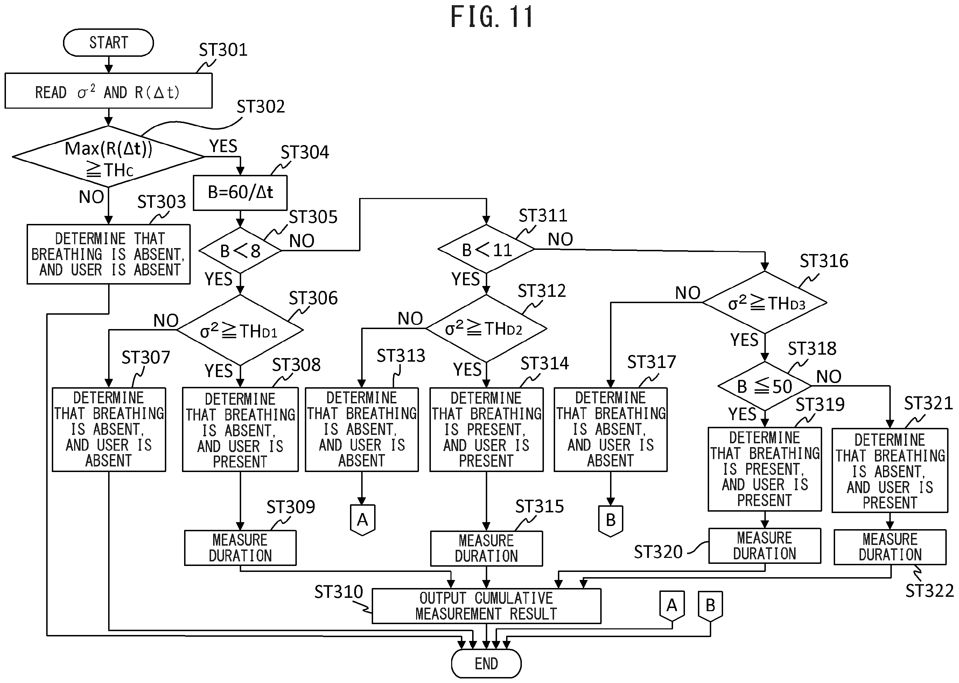

Fig. 11 is a flowchart illustrating exemplary respiratory monitoring processing, based on the respiratory information data.

The respiratory monitoring processing illustrated in Fig. 11 is performed by the microcomputer unit 7 in accordance with the computer program stored in the storage unit 71 in advance. The respiratory monitoring processing is performed once every certain duration (e.g., every 15 seconds), and updated after the elapse of the duration.

The calculation unit 725 reads the variance $\sigma^2$ and the autocorrelation coefficient $R(\Delta t)$ of the respiratory information data f(t) from the storage unit (ST301). The determination unit 726 determines whether the maximum value of the autocorrelation coefficient $R(\Delta t)$ is equal to or larger than a predetermined threshold $TH_C$ (ST302). When the maximum value of the autocorrelation coefficient $R(\Delta t)$ is smaller than the predetermined threshold $TH_C$ (NO in ST302), the determination unit 726 determines that breathing is absent, and the user of the oxygen supply device is absent (ST303), and ends the process.

When the maximum value of the autocorrelation coefficient $R(\Delta t)$ is equal to or larger than the predetermined threshold $TH_C$ (YES in ST302), the calculation unit 725 estimates a respiratory rate B per minute (bpm) = 60 seconds/$\Delta t$ using, as a respiratory interval, a time $\Delta t$ in which the maximum value of the autocorrelation coefficient $R(\Delta t)$ is obtained (ST304).

The determination unit 726 then determines whether the respiratory rate B is less than eight breaths (ST305). When the respiratory rate B is less than eight breaths (YES in ST305), the determination unit 726 determines whether the variance $\sigma^2$ is equal to or higher than a first threshold $TH_{D1}$ (ST306).

When the variance $\sigma^2$ is lower than the first threshold $TH_{D1}$ (NO in ST306), the determination unit 726 determines that breathing is absent, and the user is absent (ST307), and ends the process. When the variance $\sigma^2$ is equal to or higher than the first threshold $TH_{D1}$ (YES in ST306), the determination unit 726 determines that breathing is absent, and the user is present (ST308). The measurement unit 727 measures the duration in which the user of the oxygen supply device 1 has been determined to be present (ST309). The measured duration, for example, is defined as a predetermined monitoring duration. The output unit 728 outputs a cumulative measurement result obtained by adding the measured duration (ST310), and ends the process.

When the respiratory rate B is eight breaths or more (NO in ST305), the determination unit 726 further determines whether the respiratory rate B is less than 11 breaths (ST311). When the respiratory rate is less than 11 breaths (YES in ST311), the determination unit 726 determines whether the variance $\sigma^2$ is equal to or higher than a second threshold $TH_{D2}$ (ST312).

When the variance $\sigma^2$ is lower than the second threshold $TH_{D2}$ (NO in ST312), the determination unit 726 determines that breathing is absent, and the user is absent (ST313), and ends the process. When the variance $\sigma^2$ is equal to or higher than the second threshold $TH_{D2}$ (YES in ST312), the determination unit 726 determines that breathing is present, and the user is present (ST314). The measurement unit 727 measures the duration in which the user of the oxygen supply device 1 has been determined to be present (ST315). The output unit 728 outputs a cumulative measurement result obtained by adding the measured duration (ST310), and ends the process.

When the respiratory rate B is 11 breaths or more (NO in ST311), the determination unit 726 further determines whether the variance $\sigma^2$ is equal to or higher than a third threshold $TH_{D3}$ (ST316).

When the variance $\sigma^2$ is lower than the third threshold $TH_{D3}$ (NO in ST316), the determination unit 726 determines that breathing is absent, and the user is absent (ST317), and ends the process. When the variance $\sigma^2$ is equal to or higher than the third threshold $TH_{D3}$ (YES in ST316), the determination unit 726 further determines whether the respiratory rate B is 50 breaths or less (ST318). When the respiratory rate B is 50 breaths or less (YES in ST318), the determination unit 726 determines that breathing is present, and the user is present (ST319). The measurement unit 727 measures the duration in which the user of the oxygen supply device 1 has been determined to be present (ST320). The output unit 728 outputs a cumulative measurement result obtained by adding the measured duration (ST310), and ends the process.

When the respiratory rate B is more than 50 breaths (NO in ST318), the determination unit 726 determines that breathing is absent, and the user is present (ST321). The measurement unit 727 measures the duration in which the user of the oxygen supply device 1 has been determined to be present (ST322). The output unit 728 outputs a cumulative measurement result obtained by adding the measured duration (ST310), and ends the process.

In this embodiment, the respiratory monitoring processing is performed once every certain duration (e.g., every 15 seconds), but the measured duration may be changed in association with, e.g., the respiratory monitoring result.

The display unit 8 can display a cumulative measurement result, based on the cumulative measurement result output from the output unit 728. The display unit 8 may further display the respiratory rate.

The transmission unit 9 can transmit to the exterior the cumulative measurement result output from the output unit 728. The transmission unit 9 may further transmit the respiratory rate. An example of the destination of transmission to the exterior may be a server, a handheld terminal or personal digital assistant, or a combination of a server and a personal digital assistant. The transmission unit 9 may even temporarily store the cumulative measurement result output from the output unit 728 in the storage unit 71, and transmit it to the exterior, e.g., at a certain interval or at an arbitrary timing.

The external storage unit 10 can store the cumulative measurement result output from the output unit 728 on a storage medium such as an SD card. The external storage unit 10 may further store the respiratory rate. The external storage unit 10 may even transmit the cumulative measurement result stored on the storage medium to the exterior, e.g., at a certain interval or at an arbitrary timing.

The use of the cumulative measurement result output from the output unit 728 is not limited to the above-mentioned examples, and this result can be used in various embodiments.

In this embodiment, a PSA oxygen concentration device has been taken as an example of the oxygen supply device 1 used in combination with the respiratory monitoring device 4, but as another example, an oxygen cylinder, liquid oxygen, or any other oxygen concentration device may be employed as the oxygen supply device used in combination with the respiratory monitoring device 4.

The respiratory monitoring device 4 according to this embodiment can be used in combination with an oxygen supply device to measure the substantial inhalation time of the oxygen supply device, and perform output, transmission, display, or recording.

<Monitoring Display>

The respiratory monitoring device 4 can be used in combination with an oxygen supply device to display, on the display unit 8, the estimated inhalation time that is the substantial inhalation time of the oxygen supply device, i.e., the time of the cumulative measurement result by the respiratory monitoring processing. Simultaneously displaying the operation time and the estimated inhalation time of the oxygen supply device on the display unit 8 allows the operation administrator of the oxygen supply device to monitor whether oxygen therapy is conducted in accordance with prescription to achieve appropriate use of the oxygen supply device for the patient.

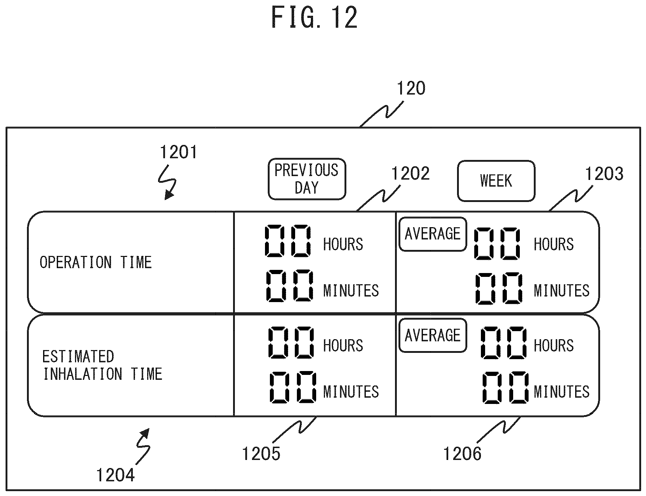

Fig. 12 is a view illustrating an exemplary respiratory monitoring display screen.

Display on a respiratory monitoring display screen 120 illustrated in Fig. 12 is performed by controlling the display unit 8 by the display control unit 729 of the microcomputer unit 7 in accordance with the computer program stored in the storage unit 71 in advance. On an upper line 1201 of the respiratory monitoring display screen 120, an operation time representing the time in which the oxygen supply device is in operation is displayed as a previous day's operation time 1202 and a week's operation time 1203. On a lower line 1204 of the respiratory monitoring display screen 120, an estimated inhalation time representing the substantial inhalation time of the oxygen supply device is displayed as a previous day's estimated inhalation time 1205 and a week's estimated inhalation time 1206.

The display control unit 729 controls the display unit 8 to display a background image, using the monitoring display image 715 stored in the storage unit 71.

Data of the operation time of the PSA oxygen concentration device 1 serving as the oxygen supply device is acquired from the PSA oxygen concentration device 1 by the operation data acquisition unit 730 of the microcomputer unit 7, and stored in the storage unit 71 as the operation data file 714. The display control unit 729 controls the display unit 8 to display the previous day's operation time 1202 and the week's operation time 1203 using the operation data file 714.

The cumulative measurement result obtained by the respiratory monitoring processing is stored in the storage unit 71 as the cumulative measurement result data file 713. The display control unit 729 controls the display unit 8 to display the previous day's estimated inhalation time 1205 and the week's estimated inhalation time 1206 using the cumulative measurement result data file 713.

The respiratory monitoring display screen 120 illustrated in Fig. 12 is merely an example, and the respiratory monitoring device 4 may display, e.g., a today's operation time and estimated inhalation time in place of the previous day's operation time and estimated inhalation time. The respiratory monitoring device 4 may use, e.g., an input unit of the microcomputer unit 7 to display a prescribed inhalation time stored in the storage unit 71 on the display unit 8, simultaneously with display of the operation time and the estimated inhalation time. Displaying the prescribed inhalation time simultaneously with display of the estimated inhalation time allows the operation administrator of the oxygen supply device to easily make a comparison with the prescribed inhalation time.

It is to be understood that those skilled in the art may make various changes, substitutions, and modifications to the present invention without departing from the spirit and scope of the present invention.

REFERENCE SIGNS LIST

1 PSA oxygen concentration device
2 Tube
3 Nasal cannula
4 Respiratory monitoring device
5 Orifice
6 Pressure sensor
7 Microcomputer unit
71 Storage unit
713 Cumulative measurement result data file
714 Operation data file
715 Monitoring display image
72 Processing unit
721 Detected data acquisition unit
722 Arithmetic operation unit
723 Estimation unit
724 Respiratory rate output unit
725 Calculation unit
726 Determination unit
727 Measurement unit
728 Output unit
729 Display control unit
730 Operation data acquisition unit
8 Display unit
9 Transmission unit
10 External storage unit

Claims

1. A respiratory monitoring device used in combination with an oxygen supply device delivering highly concentrated oxygen gas, the respiratory monitoring device comprising:

a detection unit that detects a change in breathing-related information representing at least one of a pressure, a flow rate, and a gas temperature, based on exhalation and inhalation;
a calculation unit that calculates a respiratory rate, based on the change in breathing-related information;
a determination unit that determines whether breathing is present, and whether a user of the oxygen supply device is present, based on the respiratory rate;
a measurement unit that measures a duration in which a user of an oxygen supply device has been determined to be present, based on a determination result obtained by the determination unit; and
an output unit that outputs a cumulative measurement result for the duration.

2. The respiratory monitoring device according to claim 1, wherein the determination unit determines that a user of an oxygen supply device is present unless the respiratory rate is zero or is not calculable, and further determines that breathing is present when the respiratory rate is calculated to fall within a predetermined range.

3. The respiratory monitoring device according to claim 2, wherein the predetermined range is set to 8 to 50 bpm.

4. The respiratory monitoring device according to any one of claims 1 to 3, further comprising a display unit configured to display, on an identical screen, an operation time of the oxygen supply device and the cumulative measurement result for the duration.

5. The respiratory monitoring device according to any one of claims 1 to 4, wherein the oxygen supply device comprises an oxygen concentration device.

6. The respiratory monitoring device according to claim 1, wherein
the detection unit detects a change in pressure based on exhalation and inhalation, and
the calculation unit calculates the respiratory rate, based on the change in pressure.

7. The respiratory monitoring device according to claim 6, wherein the calculation unit calculates the respiratory rate using data having a pressure variation component, independent of exhalation and inhalation, removed from the change in pressure based on the exhalation and inhalation.

8. The respiratory monitoring device according to claim 6, wherein the calculation unit estimates a pressure variation component based on an operation of the oxygen supply device, on the basis of the change in pressure based on the exhalation and inhalation, and calculates the respiratory rate using the data having the pressure variation component removed from the change in pressure based on the exhalation and inhalation.

FIG. 1

FIG. 2

# FIG. 3

# FIG. 4

# FIG. 5

FIG. 6

# FIG. 7

# FIG. 8

# FIG. 9

START

ACQUIRE PRESSURE DATA AS:Y(t) — ST101

CALCULATE AVERAGE
CORRESPONDING
TO N PERIODS AS:
$$X(t) = \frac{\sum_{i=0}^{n} a_i Y(t - iT)}{\sum_{i=0}^{n} a_i}$$
— ST102

EXTRACT PATIENT RESPIRATORY
INFORMATION DATA AS: f(t)=Y(t)-X(t) — ST103

END

# FIG. 10

START

CALCULATE AVERAGE $\mu$ AND STANDARD DEVIATION $\sigma$ OF PATIENT RESPIRATORY INFORMATION DATA f(t) — ST201

$\sigma^2 \geqq TH_D$ — ST202

YES

NO

TIME $\Delta t = 0$ — ST203

TIME $\Delta t = \Delta t + t_0$ — ST204

CALCULATE AUTOCORRELATION COEFFICIENT OF f(t) AS: — ST205

$$R(\Delta t) = \frac{1}{(n - \Delta t)\sigma^2} \sum_{j=1}^{n - \Delta t/t_0} \{f\ t\ (\ jt_0) - \mu\}[f\{t - (jt_0 + \Delta t)\} - \mu]$$

WRITE $R(\Delta t)$ — ST206

NO

$\Delta t = nt_0$ — ST207

YES

READ $R(\Delta t)$ — ST208

Max ( $R(\Delta t)$ ) $\geqq TH_C$ — ST209

NO

YES

SET $\Delta t$ AS RESPIRATORY INTERVAL — ST210

ESTIMATE RESPIRATORY RATE FROM RESPIRATORY INTERVAL $\Delta t$ — ST211

OUTPUT RESPIRATORY RATE — ST212

OUTPUT INFORMATION INDICATING THAT RESPIRATORY RATE COMPUTATION HAS BEEN IMPOSSIBLE — ST213

END

# FIG. 11

EP 3 858 407 A1

FIG. 12

120

1201

PREVIOUS DAY    1202          WEEK    1203

OPERATION TIME

00 HOURS        AVERAGE 00 HOURS
00 MINUTES      AVERAGE 00 MINUTES

ESTIMATED
INHALATION TIME

00 HOURS        AVERAGE 00 HOURS
00 MINUTES      AVERAGE 00 MINUTES

1204        1205            1206

26

## INTERNATIONAL SEARCH REPORT

| | International application No. |
|---|---|
| | PCT/JP2019/038394 |

A. CLASSIFICATION OF SUBJECT MATTER
Int.Cl. A61M16/00(2006.01)i, A61B5/087(2006.01)i, A61M16/10(2006.01)i

According to International Patent Classification (IPC) or to both national classification and IPC

B. FIELDS SEARCHED

Minimum documentation searched (classification system followed by classification symbols)
Int.Cl. A61M16/00, A61B5/087, A61M16/10

Documentation searched other than minimum documentation to the extent that such documents are included in the fields searched

| | |
|---|---|
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 2001-286566 A (FUKUDA SANGYO CO., LTD.) 16 October 2001, paragraphs [0029]-[0044], fig. 1-12 (Family: none) | 1-8 |
| A | JP 2009-261958 A (AIRSEP CORPORATION) 12 November 2009, paragraphs [0031], [0049] & JP 2005-515864 A & US 2003/0167924 A1, paragraphs [0038], [0058] & US 2004/0149133 A1 & WO 2003/064009 A1 & EP 1485188 A1 & EP 1637209 A1 | 1-8 |

☒ Further documents are listed in the continuation of Box C.   ☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| 18 November 2019 (18.11.2019) | 03 December 2019 (03.12.2019) |

| Name and mailing address of the ISA/<br>Japan Patent Office<br>3-4-3, Kasumigaseki, Chiyoda-ku,<br>Tokyo 100-8915, Japan | Authorized officer |
|---|---|
| | Telephone No. |

Form PCT/ISA/210 (second sheet) (January 2015)

**INTERNATIONAL SEARCH REPORT**

| International application No. |
| --- |
| PCT/JP2019/038394 |

C (Continuation). DOCUMENTS CONSIDERED TO BE RELEVANT

| Category* | Citation of document, with indication, where appropriate, of the relevant passages | Relevant to claim No. |
| --- | --- | --- |
| A | JP 6-190045 A (TEIJIN LTD.) 12 July 1994, paragraphs [0014]-[0049], fig. 1-6 (Family: none) | 1-8 |
| A | JP 2001-046504 A (DAIKIN INDUSTRIES, LTD.) 20 February 2001, paragraphs [0020]-[0033], fig. 1-5 (Family: none) | 1-8 |
| A | JP 2015-085191 A (NARA MEDICAL UNIVERSITY) 07 May 2015, paragraphs [0038]-[0052], [0063]-[0066], [0080]-[0082], [0099]-[0100], [0107], fig. 1-7 (Family: none) | 7-8 |

Form PCT/ISA/210 (continuation of second sheet) (January 2015)

**REFERENCES CITED IN THE DESCRIPTION**

**Patent documents cited in the description**

- JP H6190045 A
- JP 2001286566 A
- JP H796035 A
- JP 2015085191 A
- JP 2011518016 A
- WO 2018180392 A